# EUROPEAN PATENT APPLICATION

(11) **EP 4 520 330 A1**
(43) Date of publication of application: **12.03.2025**
(21) Application number: 23799497.5
(22) Date of filing: 01.05.2023
(51) Int. Cl.: A61K 31/4545, A61K 9/06, A61K 47/06, A61K 47/34, A61P 17/00, A61P 31/12, A61P 31/22

(54) **ANILINE-DERIVATIVE-CONTAINING EXTERNAL AGENT FOR SKIN**

(30) Priority: 02.05.2022 JP 2022075963
(71) Applicant: KinoPharma, Inc., Chuo-ku, Tokyo 103-0023 (JP); Iwaki Seiyaku Co., Ltd., Tokyo 103-0023 (JP)
(72) Inventor: YAMAGUCHI, Tetsuo, Tokyo 103-0023 (JP); SAITO, Hanae, Tokyo 103-0023 (JP); AKIYAMA, Yuka, Tokyo 103-0023 (JP); AKASHI, Taiki, Tokyo 103-0023 (JP); KURIMOTO, Shota, Tokyo 103-0023 (JP)
(74) Representative: Hoffmann Eitle
(86) International application number: PCT/JP2023/017077
(87) International publication number: WO 2023/214568

(57) **Abstract**

The purpose of the present invention is to provide an external preparation for skin containing an aniline derivative as a principal agent and having high principal agent stability and high transdermal absorbability. The present invention provides an external preparation for skin comprising, as an active ingredient, a compound selected from the group consisting of an aniline derivative represented by the following formula (I): 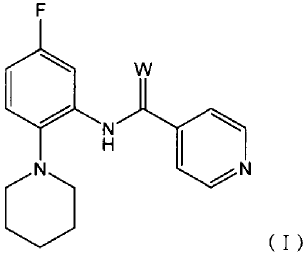 [Wherein W represents S or O]
, a pharmacologically acceptable salt thereof, and a hydrate of the same, and further comprising a monohydric higher alcohol having 16 to 20 carbon atoms or synthetic squalane.

## Description

### [Technical Field]

The present invention relates to an external preparation for skin containing an aniline derivative or a salt thereof. The present invention also relates to a transdermal delivery system for topically delivering an aniline derivative to the skin.

### [Background Art]

Human papillomavirus (HPV) is a DNA virus that belongs to the Papillomaviridae family and has a circular doublestranded DNA as its genome. HPV is transmitted by contact infection. HPV infects epithelia and mucous membranes, and the virus is replicated by expressing HPV proteins in infected host cells. In particular, it has been revealed that E6 protein and E7 protein, which are nonstructural proteins, induce abnormal cell proliferation by degrading tumor suppressor genes such as p53 gene and pRB gene of host cells, and cause various diseases such as warts such as common warts, flat warts, plantar warts, and human cervical cancer, condyloma acuminatum, pharyngeal papilloma. More than 100 types of HPV are known to date, and, depending on their carcinogenicity, classified into low-risk types, which cause benign tumors such as warts, condyloma acuminatum, papillomas, and high-risk types, which cause malignant tumors such as cervical cancer.

Treatments for human papillomavirus (HPV) warts mainly used include surgical treatments such as laser treatment (laser ablation), cryotherapy using liquid nitrogen, electrocautery, surgical excision. However, HPV viral genes tend to remain in the epidermal basal cells, resulting in frequent recurrences, which places a heavy burden on patients. BESELNA (registered trademark) cream (produced by Mochida Pharmaceutical Co., Ltd.), which contains imiquimod as an active ingredient, is used to treat condyloma acuminatum caused by HPV, but it may cause a severe inflammatory reaction, causing side effects such as erosions, ulcers, epidermal peeling, fever. Therefore, caution is being asked not to overdosing.

N-[5-fluoro-2-(1-piperidinyl)phenyl]-4-pyridinethioamide is known as a compound that exhibits antiviral activity against papillomavirus (Patent Document 1: WO2005/063293, Patent Document 2: WO2009/020198, Non-Patent Document 1: Yamamoto M et al., J Clin Invest, 2014, 124(8), 3479-3488). It is known that N-[5-fluoro-2-(1-piperidinyl)phenyl]-4-pyridinethioamide targets host cell kinase and suppresses expression of E6 and E7 genes of human papillomavirus (HPV), and stabilizes p53 gene, thereby exerting an antiviral effect. External skin therapeutic preparations for HPV warts and condyloma acuminatum using such thioamide derivatives are expected.

As a therapeutic preparation for human papillomavirus (HPV) warts, a patch agent against HPV warts characterized by containing 50 to 500 µg of N-[5-fluoro-2-(1-piperidinyl)phenyl]-4-pyridinethioamide per unit area (1 cm²) and being pasted repeatedly once a day has been reported (Patent Document 3: Japanese Patent Application Publication Laid-Open No. 2021-059500).

In addition, the above compounds have been reported to have antiviral effects against herpes simplex virus and human herpesvirus type 8 (Patent Document 2, Non-Patent Document 1, Patent Document 4: Japanese Patent Application Publication Laid-Open 2021-046360), and antiviral effects against bovine papillomavirus (Patent Document 5: WO2021/246332).

However, the above thioamide derivative is poorly soluble in water and has poor stability after dissolution, and substances exhibiting irritation such as organic solvents are not preferred for external preparations for skin, therefore, it is difficult to produce external preparations containing the thioamide derivative as an active ingredient. Furthermore, even if it can be produced as an external preparation, the active ingredient described above may not sufficiently permeate the skin and may not be absorbed sufficiently into the epidermis. Therefore, there has been no report yet of an external preparation for skin containing a thioamide derivative, which has high principal agent stability and high transdermal absorbability, and is suitable for treating human papillomavirus (HPV) warts and condyloma acuminatum.

### [Cited Prior Arts]

### [Patent Documents]

Patent Document 1: WO2005/063293
Patent Document 2: WO2009/020198
Patent Document 3: Japanese Patent Application Publication Laid-Open No. 2021-059500
Patent Document 4: Japanese Patent Application Publication Laid-Open 2021-046360
Patent Document 5: WO2021/246332

### [Non- Patent Documents]

Non-Patent Document 1: Yamamoto M et al., J Clin Invest, 2014, 124(8), 3479-3488[Summary of the Invention]

### [Technical Problem]

An object of the present invention is to provide an external preparation for skin containing an aniline derivative as an active ingredient and having high principal agent stability and high transdermal absorbability.

### [Solution to Problem]

The present inventors have extensively researched into external preparations for skin having high principal agent stability, high transdermal absorbability, and low irritation, using N-[5-fluoro-2-(1-piperidinyl)phenyl]-4-pyridinethioamide, which is an aniline derivative, and resultantly found a combination of components of external preparations for the skin that can maintain the principal agent stability and has good transdermal absorbability by using an aniline derivative together with specific absorption enhancers, leading to completion of the present invention. Furthermore, in a preferred embodiment, the external preparation for skin of the present invention is considered to have low skin irritation.

The external preparation for skin of the present invention includes the following embodiments.
[1] An external preparation for skin comprising, as an active ingredient, a compound selected from the group consisting of an aniline derivative represented by the following formula (I): 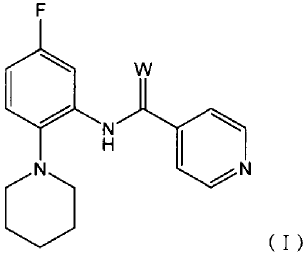 [Wherein W represents S or O]
   , a pharmacologically acceptable salt thereof, and a hydrate of the same, and further comprising a monohydric higher alcohol having 16 to 20 carbon atoms or synthetic squalane.
[2] The external preparation for skin according to [1] above, wherein the active ingredient is a compound selected from the group consisting of an aniline derivative represented by the following formula (I-a): 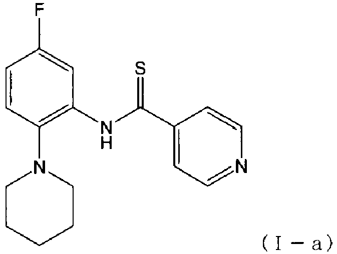 , a pharmacologically acceptable salt thereof, and a hydrate of the same.
[3] The external preparation for skin according to [1] or [2] above, wherein the monohydric higher alcohol having 16 to 20 carbon atoms is a monohydric higher alcohol having a branched structure.
[4] The external preparation for skin according to [3] above, wherein the monohydric higher alcohol having a branched structure is 2-octyldodecanol, hexyldecanol, or isostearyl alcohol.
[5] The external preparation for skin according to any one of [1] to [4] above, wherein the active ingredient is contained in an amount of about 0.5% to about 20% by weight (preferably about 0.5% to about 15% by weight, more preferably about 1% to about 10% by weight).
[6] The external preparation for skin according to any one of [1] to [5] above, wherein the monohydric higher alcohol having 16 to 20 carbon atoms or synthetic squalane is contained at about 1% to about 40% by weight (preferably about 2% to about 30% by weight, more preferably about 5% to about 20% by weight).
[7] The external preparation for skin according to any one of [1] to [6] above, wherein the weight ratio of the active ingredient to the monohydric higher alcohol having 16 to 20 carbon atoms or synthetic squalane is 1:80 to 20:1 (preferably 1:40 to 15:2, more preferably 1: 20 to 2:1).
[8] The external preparation for skin according to any one of [1] to [7] above, wherein the dosage form is an oleaginous ointment.
[9] The external preparation for skin according to [8] above, wherein the oleaginous ointment has crystals of the active ingredient dispersed in an ointment base.
[10] The external preparation for skin according to any one of [1] to [9] above, which is used for treating, improving, and/or inhibiting the progression of DNA viral diseases.
[11] The external preparation for skin according to [10] above, wherein the DNA viral disease is a disease selected from the group consisting of warts, condyloma acuminatum, vulvar intraepithelial neoplasia, anal intraepithelial neoplasia, and vaginal intraepithelial neoplasia caused by human papillomavirus (HPV); herpes labialis, genital herpes, and Kaposi varicella-like exanthema caused by herpes simplex virus; chickenpox and shingles caused by the varicella-zoster virus; Kaposi's sarcoma caused by human herpesvirus type 8; bovine papillomatosis caused by bovine papillomavirus (BPV); bovine infectious pustular vaginitis and bovine infectious balanoposthitis caused by bovine herpesvirus type 1; papillomas, inversion papilloma, and multicentric squamous cell carcinoma in situ caused by canine papillomavirus; and papillomas, inversion papilloma, and multicentric squamous cell carcinoma in situ caused by feline papillomavirus.
[12] A transdermal delivery system, comprising
   (a) an oleaginous base (ointment base);
   (b) a compound selected from the group consisting of an aniline derivative represented by the above formulae (I) and formula (I-a), a pharmacologically acceptable salt thereof and a hydrate of the same, dispersed in the base (preferably dispersed as crystals); and
   (c) a monohydric higher alcohol having 16 to 20 carbon atoms or synthetic squalane dispersed in the base.
[13] The transdermal delivery system according to [12] above, wherein the monohydric higher alcohol having 16 to 20 carbon atoms is 2-octyldodecanol, hexyldecanol, or isostearyl alcohol.
[14] The transdermal delivery system according to [12] or [13] above, wherein the weight ratio of the compound to the monohydric higher alcohol having 16 to 20 carbon atoms or synthetic squalane is 1:80 to 20:1 (preferably 1:40 to 15:2, more preferably 1: 20 to 2:1).

### [Advantageous Effect of the Invention]

According to the present invention, an external preparation for skin with high principal agent stability and high transdermal absorbability is provided. In one preferred embodiment, the external preparation for skin has low irritation.

### [Brief Description of Drawings]

FIG. 1 is a view showing an outline of the production step of an external preparation for skin (ointment) of the present invention.
FIG. 2 shows the results of administering (applying) an external preparation for skin (ointment) of the present invention to rats and confirming tissue migration into the epidermis.
FIG. 3 shows the results of administering (applying) an external preparation for skin (ointment) of the present invention to rats and confirming tissue migration into the epidermis.
FIG. 4 shows the results of administering (applying) an external preparation for skin (ointment) of the present invention to rats and confirming tissue migration into the epidermis.

### [Description of Embodiments]

The invention is described below by way of exemplary embodiments, along with preferred methods and materials that can be used in the practice of the invention. The subject matter of the present invention should not be limited to the embodiments described below. It should be noted that, unless otherwise specified herein, all technical and scientific terms used herein have the same meanings as commonly understood by those skilled in the art to which this invention pertains. Also, any materials and methods equivalent or similar to those described herein can also be used in the practice of the invention. Additionally, all publications and patents cited in the present specification in connection with the invention described in the present specification constitute a part of the present specification as indicating, for example, methods, materials, etc., that can be used in the present invention.

In the present specification, the notation "A-B" indicating a numerical range means a numerical range that indicates the endpoints A and B. The same applies to "A to B." Furthermore, in this specification, "about" is used to mean an allowance of ±10%. The weight percentages of the components described herein refer to the weight ratio of each component when the weight of the external preparation (i.e., the total formulation) is set to 100.

The external preparation for skin of the present invention contains an aniline derivative represented by the following formula (I): 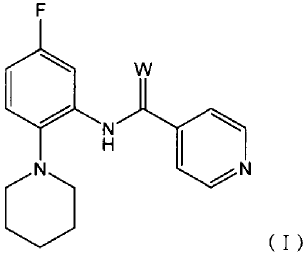 [wherein, W represents S or O]
, a pharmacologically acceptable salt thereof, or a hydrate of the same, as an active ingredient (principal agent ingredient).

The external preparation for skin of the present invention preferably contains an aniline derivative represented by the following formula (I-a): 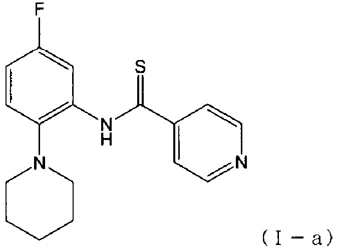 , a pharmacologically acceptable salt thereof, or a hydrate of the same, as an active ingredient (principal agent ingredient).

The term "pharmacologically acceptable salt thereof" in the present specification is not particularly limited as long as it forms a salt with the compound of the present invention represented by the above formula (I) or formula (I-a) and is pharmacologically acceptable, and examples thereof include inorganic acid salts, organic acid salts, inorganic base salts, organic base salts, and acidic or basic amino acid salts. In addition, hydrates of the same refer to compounds or salts thereof that further contain a stoichiometric or non-stoichiometric amount of water bound by non-covalent intermolecular forces.

The external preparation for skin of the present invention (hereinafter, sometimes simply referred to as external agent) contains a therapeutically effective amount of a compound, which is an aniline derivative represented by the above formula (I) or formula (I-a) or a pharmacologically acceptable salt thereof, or a hydrate of the same (hereinafter, sometimes simply referred to as active ingredient, compound, or principal agent of the present invention, and these terms have the same meaning). The therapeutically effective amount means an amount sufficient to produce at least one of the effects of treating, improving, and inhibiting the progression of a disease when the external preparation of the present invention is applied to the affected area of a subject with a disease. Alternatively, the therapeutically effective amount means an amount that can suppress the proliferation of DNA viruses in the affected area when the external preparation of the present invention is applied to the affected area of a subject with a disease. The active ingredient of the present invention is contained in an amount of, though not limited thereto, for example, about 0.5% by weight, preferably about 1.0% by weight, more preferably about 2% by weight, and further preferably about 3% by weight as the lower limit, or about 20% by weight, preferably about 15% by weight, and more preferably about 10% by weight as the upper limit, based on the total weight of the external agent. If the amount is less than 0.5% by weight, the effectiveness will be poor, while if the amount exceeds 20% by weight, the properties of the preparation may possibly not be suitable as an topical agent.

In addition to the active ingredient of the present invention, the external preparation of the present invention further contains a monohydric higher alcohol having 16 to 20 carbon atoms or synthetic squalane. The external preparation of the present invention contains a monohydric higher alcohol having 16 to 20 carbon atoms or synthetic squalane, so that when the external preparation of the present invention is applied to the skin, migration of the active ingredient of the present invention into the epidermis is promoted. That is, the monohydric higher alcohol having 16 to 20 carbon atoms or synthetic squalane in the external preparation of the present invention acts as a transdermal absorption enhancer of the active ingredient of the present invention.

The monohydric higher alcohol having 16 to 20 carbon atoms or synthetic squalane contained in the external preparation of the present invention may be a higher alcohol or synthetic squalane alone, or may be a combination of a higher alcohol and synthetic squalane. Furthermore, the higher alcohols may be used alone or in combination of two or more.

The hydrocarbon chain (aliphatic) in the monohydric higher alcohol having 16 to 20 carbon atoms may be a saturated hydrocarbon or an unsaturated hydrocarbon. The monohydric higher alcohol having 16 to 20 carbon atoms preferably has a branched-chain structure. Examples thereof include, but are not limited to, cetyl alcohol (cetanol), heptadecanol, stearyl alcohol, nonadecyl alcohol, arachyl alcohol (icosanol), palmitoyl alcohol, oleyl alcohol, 2-octyldodecanol, hexyldecanol, and isostearyl alcohol, preferably cetyl alcohol (cetanol), stearyl alcohol, oleyl alcohol, 2-octyldodecanol, hexyldecanol, and isostearyl alcohol, and more preferably 2-octyldodecanol, hexyldecanol, and isostearyl alcohol.

2-Octyldodecanol is a higher alcohol having a monohydric branched structure with 20 carbon atoms and a molecular weight of 298.5, and having the following structural formula.

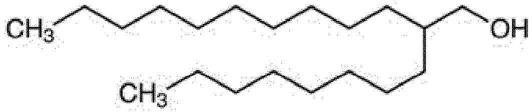

Hexyldecanol is a higher alcohol having a monohydric branched structure with 16 carbon atoms and a molecular weight of 242.44, and having the following structural formula.

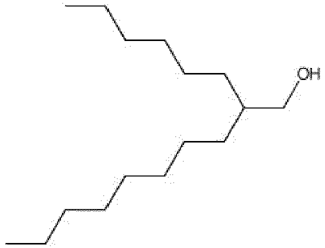

Isostearyl alcohol is a higher alcohol having a monohydric branched structure with 18 carbon atoms and a molecular weight of 270.49, and having the following structural formula.

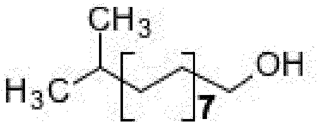

Synthetic squalane is a higher fatty acid with 30 carbon atoms and a molecular weight of 410.73, and having the following structural formula.

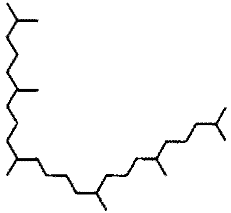

The external preparation of the present invention contains a monohydric higher alcohol having 16 to 20 carbon atoms or synthetic squalane in an amount of, for example, about 2% by weight, preferably about 3% by weight, more preferably about 5% by weight as the lower limit, or about 40% by weight, preferably about 30% by weight, more preferably about 20% by weight as the upper limit, based on the total amount of the external preparation. If the content is less than about 2% by weight, the transdermal absorbability of the active ingredient will be insufficient, while if it is more than about 40% by weight, precipitation of crystals will be noticeable and formulation will become difficult.

The external preparation of the present invention is characterized by containing a monohydric higher alcohol having 16 to 20 carbon atoms or synthetic squalane (hereinafter, sometimes simply referred to as absorption enhancer of the present invention), in addition to the active ingredient which is an aniline derivative, and the blending ratio of the active ingredient and the absorption enhancer is, for example, 1:80 to 20:1, preferably 1:40 to 15:2, more preferably 1:20 to 2:1, further preferably 1:10 to 2:1, and most preferably 1:5 to 1:1. By adjusting the blending ratio within these ranges, good transdermal absorbability of the active ingredient can be achieved, and formulation as an external preparation for skin becomes easy.

The external preparation of the present invention is not particularly limited as long as it can be applied (coated) to the skin, but is preferably an oleaginous ointment using an oleaginous base, and more preferably a dispersion-type ointment in which the active ingredient is almost uniformly dispersed in the ointment base. The aniline derivative, which is the active ingredient of the present invention, is poorly soluble and unstable when dissolved in water, so it is preferable to use an oleaginous base, and further, a crystal dispersion-type ointment dispersed in crystal state makes the active ingredient more stable and thus is more preferable. The crystal dispersion-type ointment has an advantage of being easy to prepare, but has a disadvantage of being poor in drug absorption. In the present invention, the aniline derivative, which is the active ingredient, is used in combination with a monohydric higher alcohol having 16 to 20 carbon atoms or synthetic squalane, thereby overcoming these disadvantages and providing an external preparation for skin, particularly an ointment, which has good principal agent stability and transdermal absorbability.

The external preparation for skin of the present invention in the form of an ointment will be described below. However, the present invention is not limited thereto.

When producing the external preparation of the present invention as an oleaginous ointment, it is preferable that it contains 30% by weight or more of an oleaginous base. The content of the oleaginous base in the oleaginous ointment is more preferably 40 to 90% by weight, still more preferably 50 to 90% by weight, even more preferably 60 to 90% by weight. The content of the oleaginous base herein referred to means the total content of petrolatum and other oleaginous bases exemplified below, not including the content of the monohydric higher alcohol having 16 to 20 carbon atoms or synthetic squalane functioning as the absorption enhancer in the present invention.

The oleaginous base used in the present invention can be one that adheres to the skin, retains the active ingredient on the skin for a long time, is easy to apply, does not irritate the skin, and does not affect the active ingredient stability. In the present invention, known oleaginous bases (ointment bases) can be used, and petrolatum is preferably mentioned. The petrolatum in the present invention may be either yellow petrolatum or white petrolatum in the Japanese Pharmacopoeia, but white petrolatum is preferred. Further, examples of the oleaginous base include natural wax, petroleum wax, other hydrocarbons, and the like. Examples of natural wax include beeswax (unbleached beeswax, non-chemically bleached white beeswax, chemically bleached white beeswax, etc.), carnauba wax, and the like. Examples of petroleum wax include paraffin, microcrystalline wax, and the like. Examples of other hydrocarbons include fatty acids, fatty acid esters, polyhydric alcohol fatty acid esters, higher alcohols, hydrocarbons, silicone oils, liquid paraffin, and the like. Preferred examples include hydrocarbons selected from the group consisting of paraffin, wax, and beeswax.

The ointment base in the present invention may be used alone or in combination of two or more. For example, a mixture of petrolatum and other oils may be used. The other oils that may be used may be in any of solid, semi-solid, and liquid forms. A monohydric higher alcohol having 16 to 20 carbon atoms contained in the external preparation of the present invention may also be used as the other oil referred to here.

The external preparation for skin of the present invention can also be prepared by mixing one or more additives in addition to an aniline derivative. The additives added as necessary include absorption enhancers other than those mentioned above and so on, such as water (purified water, hot spring water, deep sea water, etc.), alcohols, oils, surfactants, metal soaps, gelling agents, powders, alcohols, water-soluble polymers, film forming agents, resins, ultraviolet protection agents, clathrate compounds, antibacterial agents, fragrances, deodorants, salts, pH adjusters, antioxidants (stabilizers), refreshing agents, animal/microbial-derived extracts, plant extracts, blood circulation promoters, astringents, antiseborrheic agents, whitening agents, anti-inflammatory agents, active oxygen scavengers, cell activators, humectants, chelating agents, keratolytic agents, enzymes, hormones, vitamins, and higher alkenoic acids (e.g., oleic acid, etc.), that are commonly used in the formulation of skin cosmetics and external pharmacologicals. The ointment can be prepared according to a conventional method, and the blending amount of the additive can also be determined according to a conventional method within a range that does not impair the effects of the present invention.

The ointment of the present invention may further contain other agents effective against skin diseases. For example, anti-inflammatory agents such as glycyrrhetinic acid, antihistamine agents such as chlorpheniramine maleate, herbal ingredients such as Arnica Tincture, etc. can be mentioned, and other drugs effective for skin diseases can be blended to the extent not impairing the effects of the present invention.

The ointment of the present invention has a structure in which an aniline derivative as an active ingredient is dispersed in the ointment base together with an absorption enhancer of the present invention, and preferably has a structure in which an aniline derivative is dispersed in the form of crystal in the ointment base. The ointment of the present invention having such a structure can also be produced according to a general ointment producing method. For example, (1) an oil phase is prepared by uniformly mixing base components (white petrolatum, white beeswax, higher alcohol), if necessary base components and separation inhibitors such as surfactants and fatty acid esters under heating and stirring. Then, (2) a drug, or if necessary a product obtained by uniformly mixing a drug and base components (liquid paraffin, white petrolatum) under heating and stirring is added to the oil phase and mixed, to obtain a desired ointment. If necessary, other additives (pH adjusters, etc.) are added along with a drug. For stirring and mixing, for example, a homo mixer, a paddle mixer, a combination thereof, etc. can be used. When using multiple types of ointment bases, they can be mixed in advance.

The ointment of the present invention can be used by being sealed in any available tube (e.g., aluminum tube, aluminum laminate tube, resin (e.g., polyethylene) tube), as well as in a plastic jar container, a glass container, etc.

The aniline derivative, which is the active ingredient of the present invention, has antiviral activity against DNA viruses. Therefore, the external preparation for skin (particularly the ointment) of the present invention can be used for treating, improving, and/or inhibiting the progression of DNA viral diseases. Examples of viruses against which the active ingredient of the present invention shows antiviral activity include, but are not limited to, human adenovirus, hepatitis B virus, human herpes virus (e.g., herpes simplex virus, cytomegalovirus, EB virus, human herpes virus type 8, varicella zoster virus), smallpox virus, polyoma virus, bovine herpes virus (e.g., bovine herpes virus type 1), human papilloma virus (HPV), bovine papilloma virus, canine papilloma virus, and feline papilloma virus. Preferred are HPV, herpes simplex virus, human herpes virus type 8, bovine papilloma virus, and varicella zoster virus, and more preferred is HPV.

The external preparation for skin (particularly, ointment) of the present invention is used for diseases caused by, but not limited to, human papillomavirus (HPV), human herpes virus, bovine papilloma virus, bovine herpes virus, canine papilloma virus, or feline papilloma virus. For human diseases, the external preparation for skin of the present invention is preferably used for diseases caused by HPV, such as warts, condyloma acuminatum, vulvar intraepithelial tumor, anal intraepithelial tumor, or vaginal intraepithelial tumor; herpes labialis, genital herpes, and Kaposi varicella-like eruption caused by the herpes simplex virus; Kaposi's sarcoma caused by human herpesvirus type 8; chickenpox and shingles caused by varicella-zoster virus; and the like, and more preferably for diseases caused by HPV. The external preparation for skin of the present invention is particularly preferably used for warts or condyloma acuminatum caused by HPV. For bovine diseases, the external preparation for skin of the present invention is preferably used for diseases caused by bovine papillomavirus, such as bovine papillomatosis, diseases caused by bovine herpesvirus type 1, such as bovine infectious rhinotracheitis and bovine infectious pustular vaginitis, and the like. For canine diseases, the external preparation for skin of the present invention is preferably used for papilloma, inversion papilloma, and multicentric intraepithelial squamous cell carcinoma, etc., caused by canine papillomavirus. For feline diseases, the external preparation for skin of the present invention is preferably used for papilloma, inversion papilloma, and multicentric intraepithelial squamous cell carcinoma, etc., caused by feline papillomavirus.

The subject to which the external preparation for skin (particularly, ointment) of the present invention can be applied is not limited as long as it is a mammal that develops a disease caused by a DNA-type virus. Examples of the subject include, but not limited to, humans, monkeys, mice, rats, dogs, cows, horses, pigs, sheep, goats, cats, rabbits, hamsters, and guinea pigs, preferably humans, dogs, cows, cats, and rabbits, further preferably humans and cows, and particularly preferably humans. Then, the subject is preferably a mammal that develops a disease caused by the virus mentioned above, more preferably a mammal that develops a disease caused by a papillomavirus or human herpesvirus, and particularly preferably a mammal that develops a disease caused by a human papillomavirus.

The external preparation for skin (particularly, ointment) of the present invention can be used as an antiviral agent on affected areas of the subject's skin. The external preparation for skin of the present invention is applied in an appropriate amount to the affected area once to several times a day.

The external preparation for skin (particularly, ointment) of the present invention can also be used for preventing recurrence, by applying on the affected area of the skin subjected to treatments including surgical treatments such as laser treatment (laser ablation), cryotherapy using liquid nitrogen, electrocautery, surgical excision.

### [Examples]

Hereinafter, the present invention will be specifically explained with reference to examples, but the present invention is not limited to the following examples.

### (Example 1) Production of ointment

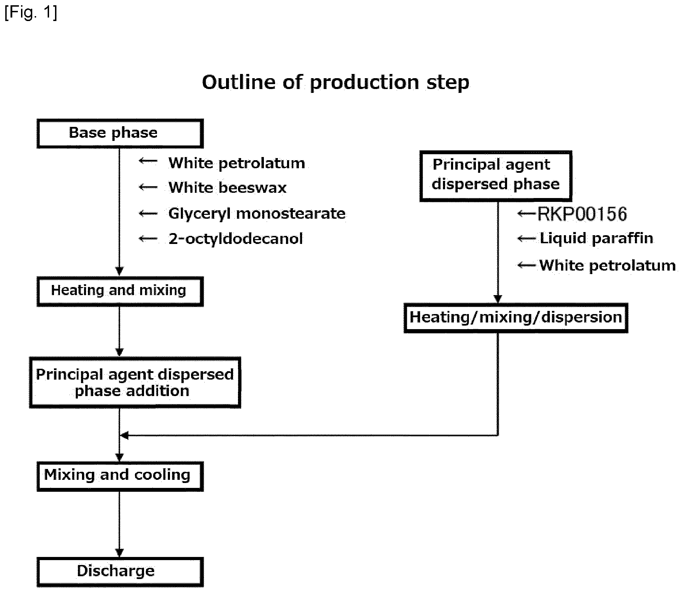

The production step of the ointment is outlined in Fig.1 The monohydric higher alcohol 2-octyldodecanol and synthetic squalane are additives, and RKP00156 is the principal agent.

### (1) Preparation of base phase

The weighed white petrolatum, white beeswax, glyceryl monostearate, and monohydric higher alcohol or synthetic squalane were stirred with a paddle and a homogenizer in an emulsifier while heating to prepare a base phase.

### (2) Preparation of principal agent dispersed phase

As a principal agent, a compound represented by the following formula (I-a) (Compound I-a): 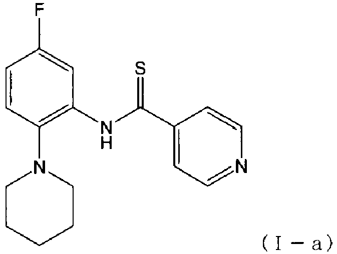 was used (Hereinafter, the above compound may sometimes be referred to as RKP00156). The principal agent, liquid paraffin, and white petrolatum were uniformly mixed while heating to prepare a principal agent dispersed phase. Dispersion of the principal agent was visually confirmed.

### (3) Preparation of ointment

The principal agent dispersed phase was added to the base phase, and when the content became uniform, it was gradually cooled to prepare an ointment before being filled into a container.

Through the above steps, ointments (Examples 1 to 7) containing 5% of the principal agent (Compound I-a) and having the compositions shown in Table 1 below were produced. The monohydric higher alcohols used in Examples 1 to 6 are as follows. Monohydric higher alcohols having 16 to 20 carbon atoms are Examples 2 to 4.
Example 1: Butyl octanol (number of carbon atoms: 12, molecular weight 186.3)
Example 2: 2-octyldodecanol (number of carbon atoms: 20, molecular weight 298.5)
Example 3: Hexyldecanol (number of carbon atoms: 16, molecular weight 242.44)
Example 4: Isostearyl alcohol (number of carbon atoms: 18, molecular weight 270.49)
Example 5: Decyltetradecanol (number of carbon atoms: 24, molecular weight 354.66)
Example 6: Dodecylhexadecanol (number of carbon atoms: 28, molecular weight 410.76)

**[Table 1]**

| **Ingredient** | **Lot/Ingredient amount (w/w%)** | | | | | | |
|---|---|---|---|---|---|---|---|
| | **Example 1** | **Example 2** | **Example 3** | **Example 4** | **Example 5** | **Example 6** | **Example 7** |
| **Compound I - a** | **5** | **5** | **5** | **5** | **5** | **5** | **5** |
| **White petrolatum** | **71** | **76** | **76** | **71** | **71** | **71** | **76** |
| **White beeswax** | **2** | **2** | **2** | **2** | **2** | **2** | **2** |
| **Glyceryl monostearate** | **2** | **2** | **2** | **2** | **2** | **2** | **2** |
| **Butyl octanol** | **10** | **-** | **-** | **-** | **-** | **-** | **-** |
| **2-octyldodecanol** | **-** | **5** | **-** | **-** | **-** | **-** | **-** |
| **Hexyldecanol** | **-** | **-** | **5** | **-** | **-** | **-** | **-** |
| **Isostearyl alcohol** | **-** | **-** | **-** | **10** | **-** | **-** | **-** |
| **Decyltetradecanol** | **-** | **-** | **-** | **-** | **10** | **-** | **-** |
| **Dodecylhexadecanol** | **-** | **-** | **-** | **-** | **-** | **10** | **-** |
| **Synthetic squalane** | **-** | **-** | **-** | **-** | **-** | **-** | **5** |
| **Liquid paraffin** | **10** | **10** | **10** | **10** | **10** | **10** | **10** |
| **Total** | **100** | **100** | **100** | **100** | **100** | **100** | **100** |

As comparative examples, ointments containing 5% of the principal agent (Comparative Examples 1 to 3) having the compositions shown in Table 2 below were produced in the same manner. Comparative Example 1 does not contain an absorption enhancer. Comparative Examples 2 and 3 contained medium-chain fatty acid triglyceride (Comparative Example 2) and isopropyl myristate (Comparative Example 3), which are absorption enhancers commonly used in ointments.

**[Table 2]**

| **Ingredient** | **Lot/Ingredient amount (w/w%)** | | |
|---|---|---|---|
| | **Comparative Example 1** | **Comparative Example 2** | **Comparative Example 3** |
| **Compound I - a** | **5** | **5** | **5** |
| **White petrolatum** | **81** | **76** | **76** |
| **White beeswax** | **2** | **2** | **2** |
| **Glyceryl monostearate** | **2** | **2** | **2** |
| **Medium-chain fatty acid triglyceride** | **-** | **5** | **-** |
| **Isopropyl myristate** | **-** | **-** | **5** |
| **Liquid paraffin** | **10** | **10** | **10** |
| **Total** | **100** | **100** | **100** |

### (Test Example 1) Confirmation of tissue migration into epidermis

Tissue migration of the principal agent of the ointment into the rat epidermis was confirmed as follows.

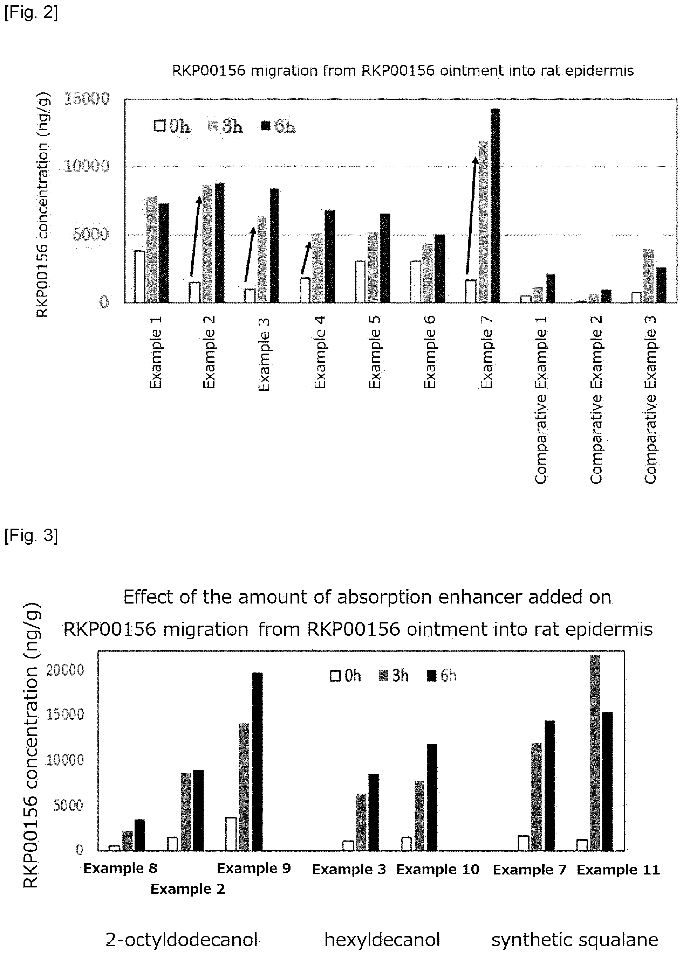

SD rats (male, 7 weeks old) were used, and the hair on the backs of the rats was shaved one day before the ointment was applied. While the animal was under anesthesia, 50 *µ*L (approximately 5 mg/cm²) of ointment was uniformly applied to an area of 3 cm × 3 cm on the shaved back skin. The ointment was applied by spreading it on the covering film in advance and pasting it on the rat's back skin. Immediately after application, the administration site was covered with a film and further fixed with an adhesive elastic bandage (performed by so-called occlusive dressing technique). At 0, 3, and 6 hours after administration, the animals were euthanized by exsanguination after blood was collected from the posterior vena cava under isoflurane anesthesia, and the adhesive elastic bandage and film were removed. The area from which the film was removed was wiped off with dry absorbent cotton, etc., then wiped twice with absorbent cotton soaked with warm water, and further wiped four times with 70% alcohol cotton. Thereafter, the applied area was shaved with a razor, tape-stripped three times, and then the skin of the applied area was collected. The collected skin was divided into epidermis and dermis, and Compound I-a, the principal agent, was extracted from each tissue, and its concentration was measured by LC/MS/MS to confirm tissue migration into each tissue. The results are shown in Figure 2.

Regarding the transdermal absorption of the active ingredient (Compound I-a), which is one of the present invention, medium chain fatty acid triglyceride (Comparative Example 2) had no effect, and isopropyl myristate (Comparative Example 3) slightly promoted transdermal absorption, but it was inadequate. In comparison, in the ointments of the present invention to which a monohydric higher alcohol or synthetic squalane was added (Examples 1 to 7), transdermal absorption of the active ingredient of the present invention was significantly promoted, and in particular, ointments containing 2-octyldodecanol, hexyldecanol, isostearyl alcohol, or synthetic squalane showed a significant transdermal absorption promoting effect.

### (Example 2) Examination of amount of transdermal absorption enhancer added (1)

The influence of the amount of absorption enhancer added on the transdermal absorption of the principal agent was confirmed as follows. Ointments (Examples 8 to 11) were produced in the same manner as in Example 1, using 2-octyldodecanol, hexyldecanol, or synthetic squalane as the absorption enhancer and changing the addition amount as shown in Table 3 below.

**[Table 3]**

| **Ingredient** | **Lot/Ingredient amount (w/w%)** | | | |
|---|---|---|---|---|
| | **Example 8** | **Example 9** | **Example 10** | **Example 11** |
| **Compound I - a** | **5** | **5** | **5** | **5** |
| **White petrolatum** | **80** | **71** | **71** | **72** |
| **White beeswax** | **2** | **2** | **2** | **2** |
| **Glyceryl monostearate** | **2** | **2** | **2** | **2** |
| **2-octyldodecanol** | **1** | **10** | **-** | **-** |
| **Hexyldecanol** | **-** | **-** | **10** | **-** |
| **Synthetic squalane** | **-** | **-** | **-** | **9** |
| **Liquid paraffin** | **10** | **10** | **10** | **10** |
| **Total** | **100** | **100** | **100** | **100** |

Using the produced ointment, the tissue migration of the principal agent in rats was examined in the same manner as in Test Example 1. The results are shown in Figure 3. The effect of promoting transdermal absorption was confirmed depending on the concentration of the absorption enhancer.

### (Example 3) Examination of amount of transdermal absorption enhancer added (2)

The amount of monohydric higher alcohol added in the production of ointments was investigated. Ointments were produced using 2-octyldodecanol as an absorption enhancer at a blending ratio of 20% to 50%, and the viscosity of the produced ointments was measured. The blending ratio and viscosity of each produced ointment are shown in Table 4 below.

**[Table 4]**

| **Ingredient** | **Lot/Ingredient amount (w/w%)** | | | | |
|---|---|---|---|---|---|
| | **Example 12** | **Example 13** | **Example 14** | **Example 15** | **Example 16** |
| **Compound I - a** | **5** | **5** | **5** | **5** | **5** |
| **White petrolatum** | **61** | **51** | **46** | **41** | **31** |
| **White beeswax** | **2** | **2** | **2** | **2** | **2** |
| **Glyceryl monostearate** | **2** | **2** | **2** | **2** | **2** |
| **2-octyldodecanol** | **20** | **30** | **35** | **40** | **50** |
| **Liquid paraffin** | **10** | **10** | **10** | **10** | **10** |
| **Total** | **100** | **100** | **100** | **100** | **100** |
| **Average viscosity (Pa · s) (n=3)** | **11.7** | **8.8** | **7.4** | **5.9** | **3.2** |

The viscosity of the ointment decreased depending on the amount of 2-octyldodecanol added. Ointments having a blending ratio up to 40% were semi-solid, while those containing 50% were highly viscous and liquid. Therefore, it was confirmed that up to 40% of the absorption enhancer can be blended. In addition, the produced ointment was applied to the skin to check the feeling of use. As a result of physical property evaluation (conditions, viscosity, degree of crystal growth, application feel) of the produced preparation, it was found that the amount of 2-octyldodecanol added was most preferably 20% or less.

### (Test Example 2) Examination of skin irritation

To rats, each 0.5 g of ointments (dose: 15, 25, 50 mg/body per day) containing 3%, 5%, and 10% of the principal agent and having the compositions shown in Table 5 below, produced in the same manner as in Example 1 (Examples 17 to 19), and a placebo ointment were administered transdermally to the back skin once a day for 90 days, as a result, no changes were observed at the administration site, and no skin irritation was observed, for any of the ointments. In addition, each 0.5 g of ointments (2 doses containing 5% and 10% of the principal agent) (Examples 18 and 19) and a placebo ointment were administered transdermally to the back skin of healthy persons twice a day at 12-hour intervals for 7 days repeatedly, as a result, no skin irritation was observed at the administration site for any of the ointments.

**[Table 5]**

| **Ingredient** | **Lot/Ingredient amount (w/w%)** | | | |
|---|---|---|---|---|
| | **Example 17** | **Example 18 (Example 2)** | **Example 19** | **Placebo** |
| **Compound I - a (RKP00156)** | **3** | **5** | **10** | **0** |
| **White petrolatum** | **78** | **76** | **71** | **81** |
| **White beeswax** | **2** | **2** | **2** | **2** |
| **Glyceryl monostearate** | **2** | **2** | **2** | **2** |
| **2-octyldodecanol** | **5** | **5** | **5** | **5** |
| **Liquid paraffin** | **10** | **10** | **10** | **10** |
| **Total** | **100** | **100** | **100** | **100** |

Furthermore, when ointments containing 5% of the principal agent having the compositions of Examples 3 and 7 shown in Table 1 were applied to the normal skin of healthy persons, no irritation was observed at the administration site 3 and 6 hours later.

### (Example 4) Examination of combination of transdermal absorption enhancers

In the same manner as in Example 1, ointments (Examples 20 and 21) containing 5% of the principal agent and having the compositions shown in Table 6 below were produced.

**[Table 6]**

| **Ingredient** | **Lot/Ingredient amount (w/w%)** | |
|---|---|---|
| | **Example 20** | **Example 21** |
| **Compound I - a** | **5** | **5** |
| **White petrolatum** | **71** | **71** |
| **White beeswax** | **2** | **2** |
| **Glyceryl monostearate** | **2** | **2** |
| **2-octyldodecanol** | **5** | **-** |
| **Hexyldecanol** | **-** | **5** |
| **Synthetic squalane** | **5** | **5** |
| **Liquid paraffin** | **10** | **10** |
| **Total** | **100** | **100** |

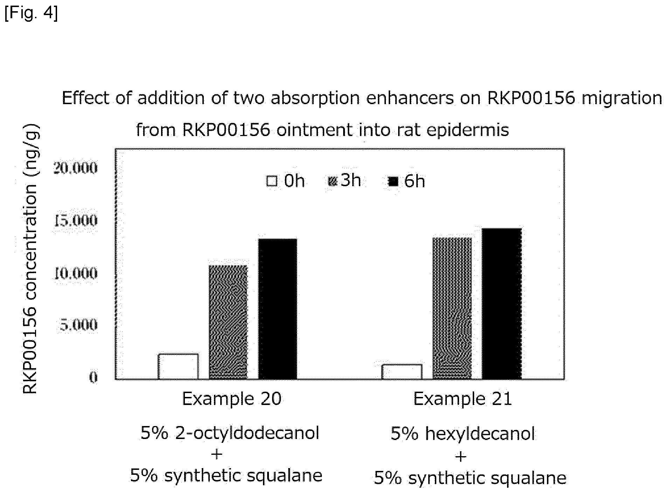

In the same manner as in Test Example 1, the migration of the principal agent from the produced ointment into the rat epidermis was confirmed. The results are shown in Figure 4. The effects of the present invention were also confirmed when monohydric higher alcohol and synthetic squalane were used in combination as absorption enhancers.

This application claims priority based on Japanese Patent Application No. 2022-75963 filed in Japan on May 2, 2022, the contents of which are incorporated herein by reference. The contents of Japanese Patent Application No. 2022-75963 are cited herein and constitute a part of the present specification.

The above detailed description is merely illustrative of the purpose and subject matter of the present invention and is not intended to limit the scope of the appended claims. Various modifications and substitutions to the described embodiments will be apparent to those skilled in the art from the teachings provided herein without departing from the scope of the appended claims.

### [Industrial Applicability]

According to the present invention, an external preparation for skin containing an aniline derivative as a principal agent has been provided. The external preparation for skin of the present invention maintains the principal agent stability and has good transdermal absorbability.

## Claims

1. An external preparation for skin comprising, as an active ingredient, a compound selected from the group consisting of an aniline derivative represented by the following formula (I): 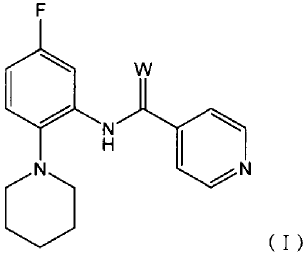 [Wherein W represents S or O]
, a pharmacologically acceptable salt thereof, and a hydrate of the same, and further comprising a monohydric higher alcohol having 16 to 20 carbon atoms or synthetic squalane.

2. The external preparation for skin according to claim 1, wherein the active ingredient is a compound selected from the group consisting of an aniline derivative represented by the following formula (I-a): 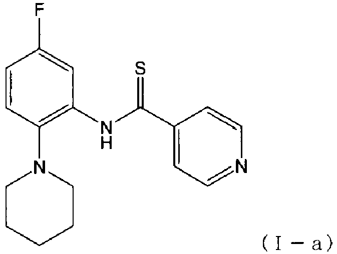 , a pharmacologically acceptable salt thereof, and a hydrate of the same.

3. The external preparation for skin according to claim 2, wherein the monohydric higher alcohol having 16 to 20 carbon atoms is a monohydric higher alcohol having a branched structure.

4. The external preparation for skin according to claim 3, wherein the monohydric higher alcohol having a branched structure is 2-octyldodecanol, hexyldecanol, or isostearyl alcohol.

5. The external preparation for skin according to any one of claims 1 to 4, wherein the active ingredient is contained in an amount of about 0.5% to about 20% by weight.

6. The external preparation for skin according to any one of claims 1 to 4, wherein the monohydric higher alcohol having 16 to 20 carbon atoms or synthetic squalane is contained at about 1% to about 40% by weight.

7. The external preparation for skin according to any one of claims 1 to 4, wherein the weight ratio of the active ingredient to the monohydric higher alcohol having 16 to 20 carbon atoms or synthetic squalane is 1:80 to 20:1.

8. The external preparation for skin according to any one of claims 1 to 4, wherein the dosage form is an oleaginous ointment.

9. The external preparation for skin according to claim 8, wherein the oleaginous ointment has crystals of the active ingredient dispersed in an ointment base.

10. The external preparation for skin according to any one of claims 1 to 4, which is used for treating, improving, and/or inhibiting the progression of DNA viral diseases.

11. The external preparation for skin according to claim 10, wherein the DNA viral disease is a disease selected from the group consisting of warts, condyloma acuminatum, vulvar intraepithelial neoplasia, anal intraepithelial neoplasia, and vaginal intraepithelial neoplasia caused by human papillomavirus (HPV); herpes labialis, genital herpes, and Kaposi varicella-like exanthema caused by herpes simplex virus; chickenpox and shingles caused by the varicella-zoster virus; Kaposi's sarcoma caused by human herpesvirus type 8; bovine papillomatosis caused by bovine papillomavirus (BPV).
